# EUROPÄISCHE PATENTANMELDUNG

(11) **EP 4 725 431 A1**
(43) Veröffentlichungstag der Anmeldung: **15.04.2026**
(21) Anmeldenummer: 24205269.4
(22) Anmeldetag: 08.10.2024
(51) Int. Cl.: A61B 18/12, A61B 5/00, A61B 18/04, A61B 18/00, A61B 34/10

(54) **KI-BASIERTER GENERATOR FÜR EIN CHIRURGISCHES INSTRUMENT**

(71) Anmelder: Erbe Elektromedizin GmbH, 72072 Tübingen (DE)
(72) Erfinder: Brandt, Tjark, 72127 Kusterdingen (DE); Keller, Sandra, 72406 Bisingen (DE); Nagel, Sebastian, 71083 Herrenberg (DE)
(74) Vertreter: Rüger Abel Patentanwälte PartGmbB

(57) **Zusammenfassung**

Mit dem erfindungsgemäßen Generator (10) kann die Behandlung von biologischem Gewebe mittels elektrochirurgischer Instrumente, insbesondere mittels Argon-Plasma-Sonden zuverlässig durchgeführt werden, ohne dabei auf die persönliche Geschicklichkeit eines Behandlers angewiesen zu sein. Mit Hilfe kameraunterstützter Messwertegewinnung wird eine Vielzahl von Probebehandlungen von Gewebepräparaten durchgeführt und auf dieser Basis ein Trainingsdatensatz erzeugt. Aus dem Trainingsdatensatz wird durch maschinelles Lernen ein Steuerdatensatz erzeugt, der im späteren Einsatz ein im Operationssaal stehendes Gerät (10) ohne Zuhilfenahme einer Kamerabeobachtung des Operationsgebiets steuert. Es werden lediglich die typischen Muster von Sensordaten ausgewertet, die in den kameraüberwachten Trainingssitzungen bestimmten Gewebeeffekten zugeordnet waren.

## Beschreibung

Die Erfindung betrifft einen KI-Generator zum Betrieb eines chirurgischen Instruments, wobei der Generator ein auf künstlicher Intelligenz beruhendes bzw. künstliche Intelligenz enthaltendes Steuermodul enthält.

Elektrochirurgische Generatoren dienen zur Versorgung elektrochirurgischer Instrumente mit Strom sowie, falls erforderlich, weiteren Betriebsmedien, wie beispielsweise Fluiden, zum Beispiel Argon, CO₂, NaCl-Lösung und so weiter. Dabei kommt insbesondere der Steuerung des elektrischen Generators besondere Bedeutung zu, weil mit diesem der Energieeintrag in biologisches Gewebe und somit der eintretende chirurgische Effekt unmittelbar steuerbar sind. Allerdings sind die Zusammenhänge zwischen den elektrischen Strom charakterisierenden Größen, wie Frequenz, Spannung, Stromstärke, Leistung, Modulationsart, Crestfaktor, sowie auch den elektrischen Größen der durch das biologische Gewebe gebildeten Last, wie beispielsweise Impedanz, und dem erzielbaren chirurgischen Effekt, unübersichtlich. Deswegen sind in der Vergangenheit verschiedene Ansätze versucht worden, Konzepte der künstlichen Intelligenz zur Generatorsteuerung heranzuziehen. Dazu beschreibt die US 2023/ 0 071 343 A1 ein computerimplementiertes Verfahren, das für Trainingszwecke sowohl elektrische Größen, Schaltzustände eines Instruments und von einer Kamera erhaltene Bilddaten nutzt. Auch bei der späteren Anwendung eines entsprechend trainierten Moduls nutzt dieses Modul aktuell erfasste elektrische Größen und zusätzlich Bilddaten, um abzuschätzen, ob die aktuellen Einstellungen zu einer erfolgreichen Anwendung führen.

Aus der US 2020/0265309 A1 ist ein Algorithmus zur Abschätzung von Gewebeparametern bekannt, der auf maschinellem Lernen beruht. Anhand von elektrischen Parametern wird ein Gewebeparameter bestimmt, der dafür genutzt wird, die Energieabgabe zu steuern.

Die DE 10 2020 105 835 A1 offenbart eine Einrichtung zur Durchführung endoskopischer Eingriffe, wobei zu dieser Einrichtung eine optische Aufnahmevorrichtung gehört, deren Sichtfeld auf das mittels der HF-Elektrode behandelte oder zu behandelnde Gewebe ausgerichtet ist. Vor oder während der Behandlung des Gewebes wird anhand von optischen Messsignalen der Aufnahmevorrichtung eine Klassifizierung des Gewebetyps des Gewebes im Bereich der HF-Elektrode durchgeführt und anhand des Ergebnisses der optischen Klassifizierung ein zu dem erkannten Gewebetyp passender HF-Mode eingestellt.

Weiterer Stand der Technik wird durch die DE 10 2021 101 410 A1**,** die US 2023/0420032 A1**,** die EP 4 134 029 A1 und die EP 3 541 313 gebildet.

Bei der elektrochirurgischen Behandlung von Gewebe, insbesondere bei der Argon-Plasma-Koagulation, benötigt der ausführende Behandler eine erhebliche Erfahrung, um schon während der Vornahme des Eingriffs das später sichtbare Resultat, das heißt, den am Gewebe erreichten Effekt einschätzen zu können. Das auf das Gewebe einwirkende Plasma emittiert selbst Licht und kann somit Blendeffekte hervorrufen. Außerdem kann das emittierte Licht aufgrund seiner spektralen Zusammensetzung eine rein visuelle Beurteilung des erzielten Resultats erschweren. Auch kann Rauchbildung, Dampfbildung oder auch eine teilweise Verdeckung des Einwirkungsorts durch das Instrument oder Gewebeteile die Beurteilung des Resultats während der Behandlung erschweren.

Davon ausgehend ist es Aufgabe der Erfindung, einen Generator anzugeben, mit dem ein Instrument so betreibbar ist, dass ein gewünschter Effekt am Gewebe unabhängig von der visuellen Beobachtung durch den Behandler und weitgehend vom persönlichen Geschick des Behandlers erzielbar ist.

Diese Aufgabe wird mit dem Generator nach Anspruch 1 gelöst:
Der erfindungsgemäße Generator enthält ein Steuermodul, das mit Sensoren für elektrische Größen verbunden ist. Die elektrischen Größen sind vorwiegend und vorzugsweise ausschließlich aus dem Strom und der Spannung abgeleitet, mit dem das an den Generator angeschlossene elektrische Instrument gespeist wird. Die elektrischen Größen können von Sensoren geliefert sein, beispielsweise Stromsensoren, Spannungssensoren, Sensoren zur Bestimmung des Leistungsfaktors, Sensoren zur Bestimmung der Nichtlinearität der angeschlossenen Last und so weiter. Das Steuermodul für den Betrieb des Generators empfängt jedoch ausschließlich elektrische Größen, nicht aber Bilddaten, die beispielsweise von einer Kamera herrühren könnten. Vielmehr kann das Steuermodul bei der Bewertung des Effektes der chirurgischen Anwendung am Patienten "blind" sein. Das Steuermodul arbeitet dabei auf Basis eines KI-Moduls, der in einem Trainingsablauf einen Steuerdatensatz erzeugt. Während des Trainings fließen die schon genannten elektrischen Größen in die Trainingsdaten ein, die später auch bei der Anwendung am Patienten überwacht werden. Die Bilddaten können Einzelbilder sein, die bei Erreichung eines gewünschten Effektlevels erfasst und abgespeichert werden. Alternativ können die Bilddaten mehrere in zeitlichen Abständen gewonnene Bilder oder auch Videosequenzen umfassen. Zum Training können Bilddaten von einer externen Kamera genutzt werden, die die Behandlung und den Behandlungsfortschritt aufnimmt. Weiter zusätzlich werden während des Trainings die am biologischen Gewebe erzielten Gewebeeffekte klassifiziert und entsprechende Effektlabels zugeordnet. Dies kann manuell oder automatisiert geschehen.

Das KI-Modul ist dazu eingerichtet, aus dem Trainingsdatensatz durch maschinelles Lernen den Steuerdatensatz zu ermitteln, der anzeigt, welche elektrischen Größen oder welches Muster elektrischer Größen vorhanden sein muss, um einem gewünschten Effektlabel entsprechenden Gewebeeffekte zu erzielen. Das Muster elektrischer Größen umfasst dabei in einer ersten Ausführungsform vorgegebene Werte oder Wertebereiche für jede überwachte elektrische Größe. In einer erweiterten Ausführungsform umfasst das Muster elektrischer Größen in Zeitabständen gewonnene Einzelwerte der elektrischen Größen oder Zeitverläufe jeder überwachten elektrischen Größe. In einer weiter verfeinerten Ausführungsform umfasst das Muster die überwachten elektrischen Größen bei wenigstens zwei unterschiedlichen Leistungsabgaben des Generators. Die unterschiedlichen Leistungsabgaben können durch unterschiedliche Spannungen, Ströme, Leistungsbegrenzungen, Modulationsformen und dergleichen bewirkt werden. Unterschiedliche Modulationsformen können z.B. Dauerstrich (CW), Amplitudenmodulation, Puls/Pause-Tastung mit konstantem oder variablem Puls/Pause-Verhältnis sein. Diese verschiedenen Leistungsabgaben können unterschiedliche Modes definieren, die unterschiedliche chirurgische Effekte hervorrufen. Außerdem kann das Muster die Zeitverläufe einer oder mehrerer elektrischer Größen vor und nach dem Umschalten des Generators zwischen verschiedenen Leistungsabgaben oder Modes umfassen. Mit diesen verschiedenen Ansätzen lassen sich verschiedene Ansprüche an die Genauigkeit des Behandlungsergebnisses erfüllen.

Die beiden oben genannten Modes unterscheiden sich vorzugsweise zumindest hinsichtlich der an das Instrument abgegebenen elektrischen Leistung. Während in dem ersten Mode eine hohe Leistung abgebbar ist, ist der zweite Mode durch eine niedrige Leistung gekennzeichnet. Vorzugsweise ist die Leistung im ersten Mode so bemessen, dass sie zu einer Devitalisierung und Koagulation von biologischem Gewebe und damit verbunden zu einem zügigen Erreichen des gewünschten Gewebeeffektes führt. Diese Leistung ist vorzugsweise größer als 10 W. Die Leistung im zweiten Mode ist vorzugsweise so bemessen, dass sie keinen chirurgischen, insbesondere keinen devitalisierenden Effekt auf das Gewebe ausübt. Die Leistung kann auf Werte von 10 Watt oder weniger begrenzt sein.

Das Steuermodul mit dem KI-Modul ist vorzugsweise dazu eingerichtet, bei Erkennung eines Musters elektrischer Größen nach einer der obigen Ausführungsformen, dem ein gewünschtes Effektlabel zugeordnet ist, von dem ersten Mode in den zweiten Mode umzuschalten. Es kann dabei optional dazu eingerichtet sein, das Muster der elektrischen Größen auch nach dem Umschalten in den zweiten Mode weiterhin zu überwachen. Dies ermöglicht es insbesondere, den nichtlinearen elektrischen Eigenschaften und den zeitlich abhängigen elektrischen Eigenschaften von biologischem Gewebe Rechnung zu tragen. Beispielsweise kann sich u.a. die Gewebeimpedanz nach Einwirkung mit hoher Leistung auch im zweiten Mode mit niedriger Leistung noch verändern. Beispielsweise kann sie durch Rückfeuchtung von zuvor ausgetrockneten Gewebepartien abnehmen.

Das Steuermodul kann weiter darauf eingerichtet sein, von dem zweiten (schwachen) Mode in den ersten (starken) Mode zurückzuschalten, wenn das im zweiten Mode aufgenommene Muster elektrischer Größen einem zu geringen Effektlabel entspricht. Umgekehrt kann es auch darauf eingerichtet sein, von dem zweiten (schwachen) Mode in einen dritten Mode zu schalten, wenn das im zweiten Mode aufgenommene Muster einem zu hohen Effektlabel entspricht. An sich ist diese Situation ein unerwarteter und normal nicht vorgesehener Fall, denn Gewebeveränderungen durch elektrische Einwirkung sind weitgehend irreversibel. Der dritte Mode kann ein Abschaltmode sein, der ein noch weiteres Überschreiten des an sich gewünschten Gewebeeffekts unterbindet. Der Gewebeeffekt ist eine Veränderung des Gewebes, z.B. eine Koagulation.

Das Steuermodul kann eine Abstandsmessfunktion umfassen. Diese kann dazu dienen, die Generierung schwer oder nicht mehr interpretierbarer Daten, d.h. elektrischer Größen der Sensoren, zu unterbinden. Bei zu großem Abstand zwischen Instrument und Gewebe können aufgrund des dann (zu) großen Einflusses der elektrischen Nichtlinearität des zwischen dem Instrument (insbesondere seiner Elektrode) und dem Gewebe unterhaltenen Funkens bzw. Plasmas Signalverzerrungen auftreten, die eine sichere Signalinterpretation beeinträchtigen oder verhindern. Die Abstandsbestimmung kann zum Beispiel im Wege einer Mustererkennung erfolgen, im Rahmen derer bestimmte Muster elektrischer Größen, die auf zu lange Entladungsstrecken (Plasma) zurückgehen erkannt werden, um bspw. den Chirurgen zur Nachführung des Instruments aufzufordern.

Die Erfindung richtet sich auch auf das oben geschilderte Verfahren zum Training eines Steuermoduls und seines KI-Moduls eines elektrischen Generators und zum späteren Betrieb eines solchen Generators. Dieses Verfahren umfasst einen Trainingsablauf, im Rahmen dessen ein Trainingsdatensatz generiert wird. Dieser wird vorzugsweise mit einer festen, dem Generator vorgegebenen Einstellung gewonnen. Mit dieser Einstellung wirkt das von dem Generator gespeiste Instrument auf das Gewebe ein, wobei sich die dabei ergebenden elektrischen Größen, insbesondere deren zeitliche Verläufe und Werte sowie sich ergebende Gewebeveränderungen, erfasst werden, die mittels Kamera in Form von Bildern, Bildfolgen oder Videosequenzen aufgezeichnet werden. Die behandelten Gewebeproben werden maschinell oder manuell inspiziert und hinsichtlich der erzielten Gewebeeffekte klassifiziert. Entsprechend werden den Bildern, Bildfolgen und/oder Videosequenzen und den gewonnenen Mustern der elektrischen Größen Effektlabels zugeordnet. Aus der Gesamtheit der Muster der gewonnenen elektrischen Größen, der Bilder, Bildfolgen und/oder Videosequenzen und der zugeordneten Effektlabels (Trainingsdatensatz) wird ein Steuerdatensatz generiert, der lediglich noch einen Zusammenhang zwischen den elektrischen Größen und den Effektlabels repräsentiert. Mittels des Steuerdatensatzes kann somit ohne Zuhilfenahme eines Kamerabildes die elektrische Einwirkung auf das biologische Gewebe so gesteuert werden, dass das Behandlungsergebnis dem gewünschten Effektlabel entspricht.

Damit wird ein Generator bereitgestellt, der wie herkömmliche Generatoren eine Auswahlmöglichkeit für die gewünschte Effektstärke bei einem bestimmten Mode aufweist. Allerdings ist die Effektstärke nun nicht mehr ein der Ausgangsleistung zugeordneter Wert auf einer abstrakten Skala, beispielsweise von 1 bis 10, sondern eine Vorgabe von Behandlungsergebnissen. Beispielsweise können die Effektstärken in mehreren Stufen, beispielsweise vier Stufen (wenig Koagulation, schwache Koagulation, starke Koagulation, sehr starke Koagulation) vorgegeben sein. Wählt der Bediener schwache Koagulation, arbeitet der Generator anhand des Steuerdatensatzes so lange im ersten Mode bis schwache Koagulation erreicht ist und schaltet dann auf den zweiten Mode um, in welchem keine weitere Koagulation erreicht wird. Wählt der Bediener starke Koagulation, arbeitet der Generator zunächst ebenfalls im ersten Mode und zwar so lange bis starke Koagulation erreicht ist, wonach er in den zweiten Mode umschaltet. Damit wird das Behandlungsergebnis weitgehend unabhängig von der persönlichen Fertigkeit des Behandlers. Dieser kann immer auf dem vor ihm liegenden Gewebe den gewünschten chirurgischen Effekt erzielen, und zwar unabhängig von der subjektiven optischen Beurteilung der Umwandlungsvorgänge am Gewebe während der Behandlung.

Weitere Details, vorteilhafte Ausführungsformen der Erfindung ergeben sich aus der nachfolgenden Beschreibung und der Zeichnung. In der Zeichnung zeigen
Figur 1 den erfindungsgemäßen Generator, das angeschlossene Instrument und ein biologisches Objekt während eines chirurgischen Eingriffs, in schematisierter Darstellung,
Figur 2 ein Beispiel eines Generators zur Erzeugung eines Trainingsdatensatzes mit angeschlossener Kamera und angeschlossenem Instrument bei der Einwirkung auf ein biologisches Objekt, in schematisierter Darstellung,
Figur 2a ein weiteres Beispiel eines Generators zur Erzeugung eines Trainingsdatensatzes mit angeschlossener Kamera und angeschlossenem Instrument bei der Einwirkung auf ein biologisches Objekt, in schematisierter Darstellung,
Figur 3 Blockdarstellungen zur Veranschaulichung der Gewinnung eines Trainingsdatensatzes und daraus der Gewinnung eines Steuerdatensatzes,
Figur 4 verschiedene Muster gewonnener elektrischer Größen während der Behandlung von biologischem Gewebe als Diagramm,
Figur 5 ein Diagramm zur Veranschaulichung des Umschaltens zwischen verschiedenen Modes des Generators.

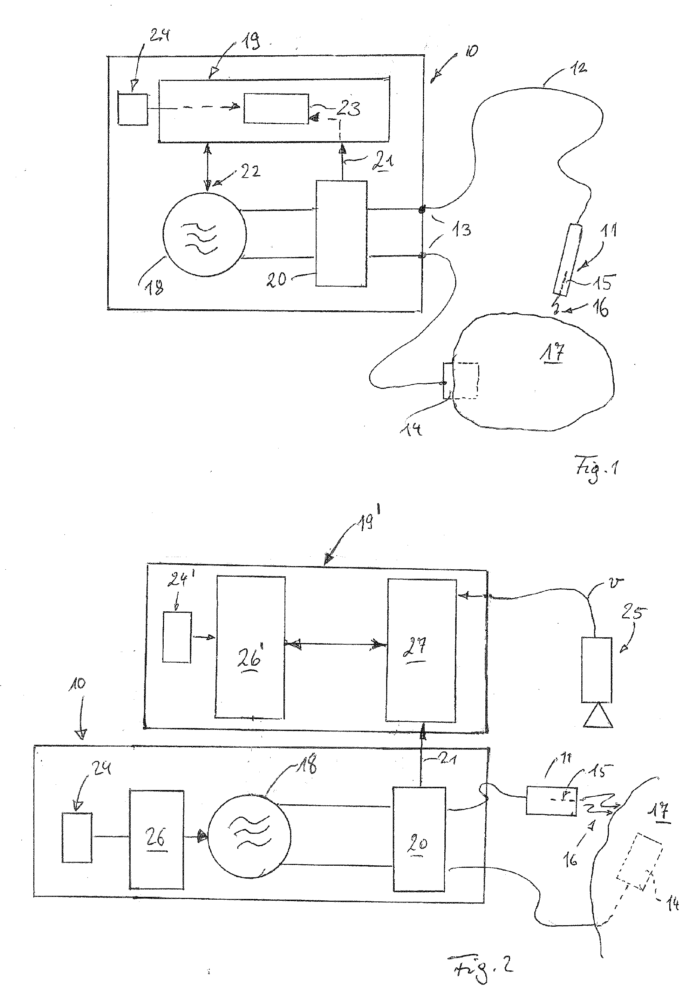

In Figur 1 ist ein erfindungsgemäßer Generator 10 veranschaulicht, an dem ein Instrument 11, insbesondere ein Argon-Plasma-Instrument, angeschlossen ist. Dieses Instrument 11 ist über ein Kabel 12 mit einem Generatorausgang 13 verbunden, an den auch eine Neutralelektrode 14 zur Rückführung des an das Instrument 11 abgegebenen Stromes angeschlossen ist. Diese Struktur gilt bei monopolaren Instrumenten. Bei bipolaren Instrumenten sind beide Pole des Ausgangs des Generatorausgangs 13 an das Instrument 11 angeschlossen.

Handelt es sich bei dem Instrument 11 spezifisch um ein Argon-Plasma-Instrument, wird dieses zusätzlich von dem Generator 10 oder einem anderen speisenden Gerät mit Gas, insbesondere Argon, versorgt, was in Figur 1 der Übersichtlichkeit wegen jedoch nicht dargestellt ist. Dabei können in den Trainingsdatensatz und damit in den Steuerungsdatensatz auch Parameter des Gasflusses einbezogen werden.

Das Instrument 11 enthält eine Elektrode 15, die in einem gasführenden, insbesondere Argon-führenden Kanal angeordnet sein kann und in Figur 1 gestrichelt dargestellt ist. Von dieser Elektrode 15 geht eine von dem Generator 10 gespeiste Plasma-Entladung 16 aus, innerhalb derer elektrischer Strom von der Elektrode 15 zu dem biologischen Gewebe 17 fließt, an welches die Neutralelektrode 14 angeschlossen ist. Dieser elektrische Strom übt eine thermische Wirkung auf das biologische Gewebe aus.

Zur Speisung des Instruments 11, d.h. zur Bereitstellung elektrischer Leistung an dem Ausgang 13, weist der Generator 10 eine Quelle 18, insbesondere eine HF-Quelle für hochfrequente elektrische Spannung auf, die von einem Steuermodul 19 steuerbar ist. Das Steuermodul 19 kann dabei insbesondere ausgewählte elektrische Größen, z.B. die Modulation, die Höhe (Amplitude) der Spannung, die Stromstärke und Ähnliches steuern.

Die Quelle 18 wird beispielsweise durch einen hochfrequent schwingenden Oszillator gebildet, der dazu eingerichtet ist, eine Spannung von mehreren 1000 Vₚₑₐₖ (Spannung Spitze zu Spitze), und eine elektrische Leistung von mehreren Watt, vorzugsweise > 10 W, beispielsweise 100 Watt, an den Ausgang 13 abzugeben. Zur Erfassung elektrischer charakteristischer Größen, wie z.B. der Spannung des Stroms, des Phasenwinkels zwischen der Spannung und dem Strom, der nichtharmonischen Verzerrung des Stroms usw. dient ein Sensorblock 20, der die gemessenen Größen an das Steuermodul 19 liefert, wie ein Pfeil 21 andeutet. Der Pfeil 21 markiert die Richtung des Informationsflusses und weist deswegen lediglich ein gepfeiltes Ende auf. Es ist aber auch möglich, die Ausführungsform so auszugestalten, dass das Steuermodul 19 gezielt Sensordaten abfragt und dazu Abfrageaufforderungen an den Sensorblock 20 übermittelt.

Das Steuermodul 19 steuert den Oszillator 18, was durch einen Pfeil 22 symbolisiert ist. Außerdem kann das Steuermodul 19 von dem Oszillator 18 Informationen erhalten, die nicht von dem an dem Gewebe 17 erzielten chirurgischen Effekt abhängig sind. Solche Information kann beispielsweise die Information über die Schwingfrequenz des Oszillators 18 oder seine Modulationsart sein (zum Beispiel Dauerstrich (CW) oder gepulst, zum Beispiel ein/aus-getastet) .

Das Steuermodul 19 enthält einen Steuerdatensatz, der durch maschinelles Lernen aus einem Trainingsdatensatz erzeugt worden ist. Der Steuerdatensatz ist dazu eingerichtet, einen Zusammenhang zwischen Effektstärken bzw. Effektstufen und elektrischen Größen herzustellen, die von dem Oszillator 18 und/oder dem Sensorblock 20 herrühren. Die entsprechenden Effektstärken bzw. Gewebeeffekte werden über eine Eingabeeinrichtung 24 vorgegeben, die ein Bestandteil des Generators 10 ist oder gesondert von diesem aufgebaut sein kann, beispielsweise durch ein mobiles Endgerät, wie zum Beispiel ein Tablet, Mobiltelefon oder dergleichen. Insbesondere kann die Eingabeeinrichtung 24 auch Teil des Instruments 11 sein.

Wie ersichtlich, beruht die Steuerung des Generators 10 allein auf der mit der Eingabeeinrichtung 24 vorgegebenen Effektstärke sowie elektrischer Größen aus dem Oszillator 18 und/oder dem Sensorblock 20. Es ist weder eine Kamera zur Inspektion des an dem biologischen Gewebe 17 erzielten Effekts vorgesehen noch erforderlich. Die mit der Eingabeeinrichtung vorgegebene Effektstärke charakterisiert, anders als bei herkömmlichen Geräten, den am biologischen Gewebe tatsächlich zu erreichenden Effekt, der beispielsweise durch einen Bräunungsgrad des Gewebes charakterisiert wird. Sobald nämlich der gewünschte Effekt erreicht ist, schaltet der Generator auf den zweiten Mode, in dem keine weitere Gewebebeeinflussung mehr stattfindet. Dies ohne dass dazu ein Kamerabild des behandelten Gewebes nötig wäre. Deswegen wird der gewünschte Effekt erreicht, aber nicht übertroffen, auch wenn der Plasmastrahl unnötig lange auf einen Gewebebereich gerichtet wird.

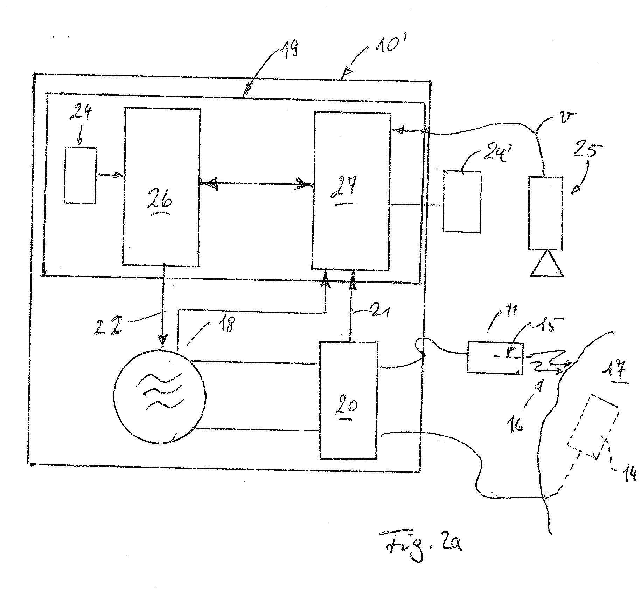

Figur 2 veranschaulicht die Gewinnung eines Trainingsdatensatzes 27, auf dessen Basis später von dem KI-Modul 23 der Steuerdatensatz erzeugt wird. Dafür ist ein separates Trainingsmodul 19' vorgesehen. Dem Trainingsmodul 19' werden zusätzlich zu den Sensorblock 20 gewonnenen Größen (siehe Pfeil 21) Bilddaten v von einer Kamera 25 zugeführt, die das Gewebe 17, insbesondere an der Stelle im Blickfeld hat, auf der die Entladung 16 einwirkt. Das Trainingsmodul 19' dient der Gewinnung eines Trainingsdatensatzes 27. Außerdem ist in dem Trainingsmodul 19` ein Trainingseingabemittel 24' vorgesehen, über das eine mit der Gewinnung des Trainingsdatensatzes 27 beauftragte Person den erreichten Effekt eingeben kann. Werden z.B. mehrere Bräunungsstufen, z.B. 10 Bräunungsstufen des Gewebes unterschieden, können sie von keiner Bräunung bis zu beginnender Verkohlung gehen. Beim Training wird in dem Trainingsdatensatz 27 dieser Bräunungsgrad als Effektlabel den Mustern der elektrischen Größen zugeordnet, die über den Sensorblock 20 gewonnen und oder direkt von dem Oszillator 18 erhalten wurden. Außerdem können über das Trainingseingabemittel 24` unterschiedliche Gewebetypen einstellbar sein. Das Trainingsmodul 19' weist außerdem einen Block 26' auf, der dazu eingerichtet ist, Ansteuersignale für einen Oszillator 18 und eine dazugehörige Leistungsabgabe des Generators 10 an das Instrument 11 zu erzeugen und/oder zu speichern. Die Ansteuersignale können verschiedenen Modes zugeordnet sein, die während der Gewinnung des Trainingsdatensatzes 27 verwendet werden. In einem ersten Mode gibt der Oszillator 18 eine hohe Leistung an das Instrument 11 ab, die auf dem Gewebe 17 einen chirurgischen Effekt, beispielsweise eine sichtbare Koagulation, erzeugt. In einem zweiten, schwächeren Mode ist die von dem Oszillator 18 an das Instrument 11 abgegebene Leistung so weit gemindert, dass die Entladung 16 keinen sichtbaren Effekt mehr auf der Oberfläche des Gewebes 17 erzielt.

Der Generator 10 weist in Figur 2 eine Eingabeeinrichtung 24 und einen Block 26 auf. Über das Eingabemittel 24 kann eine mit der Gewinnung des Trainingsdatensatzes 27 beauftragte Person nach probeweiser Einwirkung auf das Gewebe 17 mittels der Entladung 16 den erreichten Effekt eingeben kann. Der Block 26 ist dazu eingerichtet, den Oszillator 18 und somit die Leistungsabgabe des Generators 10 an das Instrument 11 zu steuern.

Figur 2a veranschaulicht ein weiteres Beispiel eines Generators 10` zur Erzeugung eines Trainingsdatensatzes. Für das in Figur 2a gezeigte Beispiel gilt das in Bezug auf Figur 2 Erläuterte mit Bezug auf die Bezugszeichen entsprechend. Das in Figur 2a gezeigte Beispiel unterscheidet sich von dem in Figur 2 gezeigten Beispiel im Wesentlichen dadurch, dass das Trainingsmodul 19' nicht von dem Generator 10' getrennt ist.

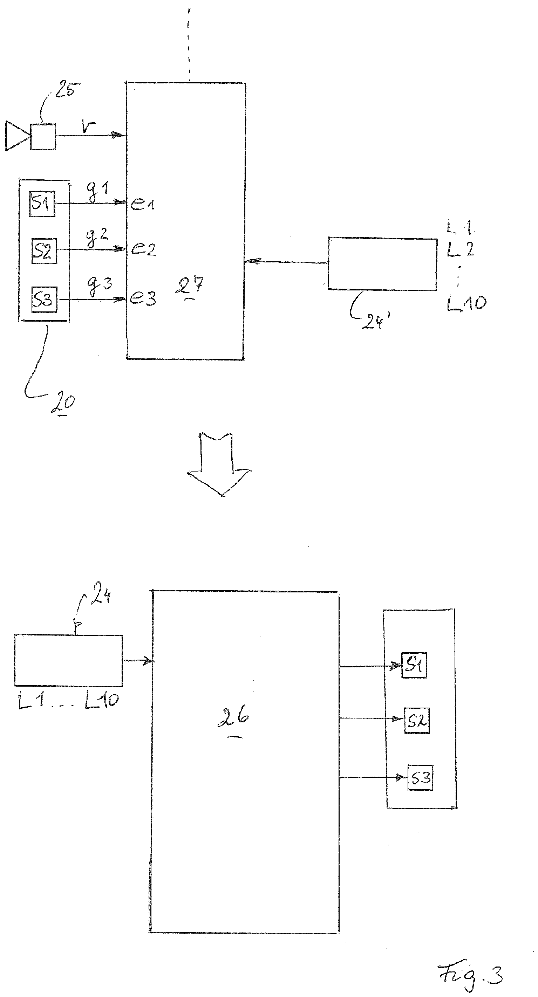

Figur 3 veranschaulicht die Gewinnung des Trainingsdatensatzes, in den sowohl Videosequenzen oder Einzelbilder der Kamera 25 als auch die Daten aus dem Sensor Block 20 einfließen. Diese Daten können die Ausgaben g1, g2, g3 eines oder mehrerer Sensoren, z.B. der Sensoren S1, S2, S3 sein, die z.B. die Größe des Stroms, die Größe des Phasenwinkels zwischen Strom und Spannung, den Crestfaktor und/oder die Nichtlinearität des Stroms kennzeichnen. Weitere Größen können ebenso gut erfasst werden. Die Vorgenannten sind lediglich beispielhaft. Es können in einem ersten Ausführungsbeispiel lediglich die von den Sensoren S1 bis S3 erfassten Größen zu Ende der Einwirkung erfasst werden. Die Größen bilden somit ein statisches Muster ohne Zeitkomponente. Jedoch ist es auch möglich, Zeitverläufe zu erfassen. Dies gilt sowohl für die Kamera 25 als auch für den Sensor Block 20, sodass die zeitlichen Verläufe der entsprechenden Größen erfasst werden. Figur 3 oben veranschaulicht dabei, dass zur Gewinnung des Trainingsdatensatzes 27 sowohl die Daten aus dem Sensor Block 20 als auch der Kamera 25 wie auch der Eingabeeinrichtung 24' zusammenfließen. Während die Daten aus der Kamera 25 und dem Sensorblock 20 den Ist-Zustand der optischen und elektrischen Größen kennzeichnen, kennzeichnet das über die Eingabeeinrichtung 24' eingegebene Effektlabel L1 ... L10 das erzielte Resultat.

Zur Gewinnung des Trainingsdatensatzes 27 wird eine Vielzahl von Musterbehandlungsvorgängen durchgeführt, bei denen an einem entsprechenden Präparat eine elektrische Einwirkung mittels der Einrichtung nach Figur 2 vorgenommen wird. Durch maschinelles Lernen wird daraus der in Figur 3 unten veranschaulichte Steuerdatensatz 26 ermittelt. Dieser erhält als Eingangsgröße über die Eingabeeinrichtung 24 den gewünschten Effekt (beispielsweise mittlere Bräunung). Aus den Informationen des Trainingsdatensatzes hat der Steuerdatensatz nun die entsprechenden Werte parat, die die Sensoren S1 bis S3 aufweisen müssen, um den gewünschten Effekt zu erzielen. Der Steuerdatensatz 26 überwacht nun die Sensoren S1 bis S4 auf die Erreichung der vorgegebenen Werte. Das Steuermodul 19 arbeitet dabei wie folgt:

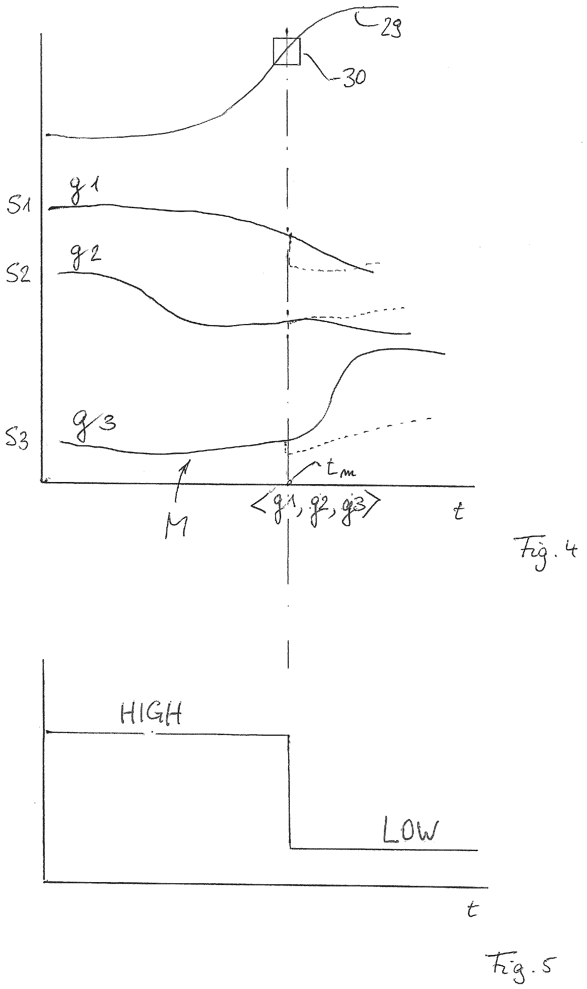

Der Oszillator 18 kann mindestens in einem ersten Mode mit hoher Leistung betrieben werden, die einen chirurgischen Effekt auf dem Gewebe 17 erzielt, sowie mit einer zweiten, niedrigeren Leistung, die keinen chirurgischen Effekt erzielt. Sowohl bei Gewinnung der Trainingsdaten mit der Einrichtung nach Figur 2 wird der Oszillator 18 zunächst in dem ersten Mode betrieben und dann abgeschaltet bzw. in den zweiten Mode überführt, wonach das Effektlabel dem erzielten Behandlungsergebnis entsprechend festgelegt wird. Bei der eigentlichen Behandlung eines Patienten mit der Einrichtung nach Figur 1 arbeitet das Steuermodul 19 nun mit dem Steuerdatensatz zu Beginn der Behandlung ebenfalls im ersten Mode. Die tatsächlich erreichte möglicherweise aber für den Chirurgen nicht gut erkennbare Umwandlung (zum Beispiel Bräunung) des Gewebes 17 ist in Figur 4 durch eine obere Kurve 29 veranschaulicht. Wie ersichtlich nimmt die Bräunung mit der Zeit zu, bis sie ein Maximum erreicht. Die entsprechenden Größen der Sensoren S1, S2, S3 sowie eventuell weiterer Sensoren können unterschiedliche Zeitverläufe aufweisen. Beispielsweise kann der Wert g1 des Sensors S1 den Strom repräsentieren, der abnehmend verlaufen kann. Der Wert g2 des Sensors S2 kann beispielsweise ein Feuchtigkeitswert sein, wobei die Gewebefeuchtigkeit mit der Zeit und mit Behandlungsfortschritt abnehmen kann. Ein dritter Wert g3 des Sensors S3 kann irgendeine andere elektrische Größe oder eine aus den elektrischen Größen errechnete, das Gewebe 17 kennzeichnete Größe sein.

Im vorliegenden Ausführungsbeispiel hat der Behandler einen Effekt, das heißt einen Bräunungsgrad vorgegeben, der in Figur 4 als Toleranzfeld in einem Kästchen 30 veranschaulicht ist. In dem Steuerdatensatz 26 ist der von dem Kästchen 30 charakterisierte Wert den charakteristischen Verläufen und somit Mustern der von den Sensoren S1, S2, S3 überwachten elektrischen Größen zugeordnet, die in Figur 4 dargestellt sind. Das Muster kann die Kombination der Größen g1, g2, g3 der Sensoren S1, S2, S3, zu einem Zeitpunkt tₘ (Messzeitpunkt) sein. Das Muster kann jedoch auch Teile der Zeitverläufe oder die gesamten Zeitverläufe der Größen der drei Sensoren S1, S2, S3 umfassen. Sobald dieses Muster erkannt wird, reduziert das Steuermodul 19 die Energieabgabe an das Instrument 11 von dem in Figur 5 veranschaulichten hohen Wert HIGH auf einen niedrigen Wert LOW.

Mit dem erfindungsgemäßen Generator 10 kann die Behandlung von biologischem Gewebe mittels elektrochirurgischer Instrumente, insbesondere mittels Argon-Plasma-Sonden zuverlässig durchgeführt werden, ohne dabei auf die persönliche Geschicklichkeit eines Behandlers angewiesen zu sein. Mit Hilfe kameraunterstützter Messwertegewinnung wird eine Vielzahl von Probebehandlungen von Gewebepräparaten durchgeführt und auf dieser Basis ein Trainingsdatensatz erzeugt. Aus dem Trainingsdatensatz wird durch maschinelles Lernen ein Steuerdatensatz erzeugt, der im späteren Einsatz ein im Operationssaal stehendes Gerät 10 ohne Zuhilfenahme einer Kamerabeobachtung des Operationsgebiets steuert. Es werden lediglich die typischen Muster von Sensordaten ausgewertet, die in den kameraüberwachten Trainingssitzungen bestimmten Gewebeeffekten zugeordnet wurden.

Mit der Erfindung ist es außerdem möglich, die thermische Wirkung auf das Gewebe sehr schnell (so schnell wie möglich) und auf kontrollierte Weise (keine Überdosierung) zu erzeugen. Dazu arbeitet das Steuermodul 19 in der Anwendungsphase mit zwei Betriebszuständen (Modes), nämlich einem Zustand mit hoher Leistung und einem Zustand mit niedriger Leistung. Der Hochleistungszustand (erster Mode) wird verwendet, um die gewünschte thermische Wirkung auf das Gewebe zu erzielen. Der Niedrigleistungszustand (zweiter Mode) hingegen ist nicht für die Erzielung einer thermischen Wirkung gedacht. Während das Plasma - das zur berührungslosen Übertragung der Leistung auf das Gewebe dient - in dem ersten Mode gezündet ist und ein Strom fließt, werden elektrische Daten (hier Spitzenspannung Up, Spitzenstrom Iₚ, Effektivspannung Uᵣₘₛ, Effektivstrom Iᵣₘₛ, Leistungsfaktor, Frequenz, Widerstand, Funkenbildung) erfasst, die als Eingangsdaten für das Steuermodul 19 dienen. Auf der Grundlage der Vorhersage des Steuermoduls 19 wird der Oszillator automatisch zwischen dem ersten Mode und dem zweiten Mode umgeschaltet, um die Gewebeschädigung sofort zu begrenzen und den gewählten Grad der Devitalisierung zu erreichen. Liegt die von dem Steuermodul 19 vorhergesagte Gewebeschädigung unter dem eingestellten Schwellenwert (gewählter Effekt), bleibt das System 18 im ersten Mode. Liegt die KI-Vorhersage über dem festgelegten Effekt, schaltet das System 18 in den zweiten Mode, um weitere thermische Schäden zu verhindern. Der zweite Mode ist dadurch gekennzeichnet, dass die abgegebene Leistung niedrig genug ist, um keine weiteren Gewebeschäden zu verursachen (hier: 10 W), aber hoch genug, um eine gültige KI-Vorhersage der erzielten elektrischen Daten innerhalb dieses Modes zu ermöglichen. Die abgegebene Leistung ist dabei so groß, um im zweiten Mode ein stabiles Plasma zu halten, um valide elektrische Daten zu sammeln, welche die Grundlage für die Entscheidung bilden, ob in den ersten Mode zu wechseln ist.

Um den Energieeintrag und damit die daraus resultierende Gewebeschädigung im zweiten Mode weiter zu reduzieren, kann die Spannung gepulst werden. Pulsieren bedeutet in diesem Fall, dass die Spannung (und der Strom) im zweiten Mode zwischen einer EIN- und einer AUS-Phase hin- und herwechseln. In der EIN-Phase wird die definierte geringe Leistung angelegt. Während der AUS-Phase wird keine Leistung an das Gewebe abgegeben. Damit wird der Gesamtenergieeintrag während des zweiten Modes deutlich reduziert, was die Devitalisierung des Gewebes in diesem Zustand weiter verringert. Die Summe der EIN-Phase und der AUS-Phase kann auf 10 ms festgelegt sein; die EIN-Phase kann von 2,4 bis 10 ms eingestellt sein. Die verbleibende Zeit ist die AUS-Phase, in der kein Strom abgegeben wird. Wenn 10 ms für die EIN-Phase gewählt wird, ist die AUS-Phase 0 ms und es findet keine Leistungsreduzierung statt, so dass der Low-Power-Zustand immer in der EIN-Phase ist. Die Regelung zwischen dem Hoch- und Niedrigleistungszustand (ersten und zweiten Mode) soll eine Überdosierung (des elektrischen Stroms) verhindern und eine reproduzierbare und damit homogene Gewebewirkung erzielen. Sobald der voreingestellte Gewebeeffekt erreicht ist, schaltet das Steuermodul 19 in einen Zustand, in dem weniger Leistung zur Verfügung steht. Mit dieser geringen Leistung wird kein nennenswerter Gewebeeffekt hervorgerufen.

Die reine Latenzzeit vom Zeitpunkt der gemessenen elektrischen Daten, über die Vorhersage des Steuermoduls 19, bis zum möglichen Umschalten der Zustände beträgt ca. 15 ms. Hinzu kommen weitere Latenzen im Millisekundenbereich, z.B. durch das Einschwingverhalten der HF-Quelle 18. Die Regelung zwischen dem Hoch- und Niedrigleistungszustand soll eine Überdosierung (des elektrischen Stroms) verhindern und eine reproduzierbare und damit homogene Gewebewirkung erzielen. Sobald der voreingestellte Gewebeeffekt erreicht ist, schaltet das Steuermodul 19 in einen Zustand, in dem weniger Leistung zur Verfügung steht. Mit dieser geringen Leistung wird kein nennenswerter Gewebeeffekt hervorgerufen.

Für das Training der KI wurden die elektrischen Daten mit dem Grad der Devitalisierung der Gewebeoberfläche gekennzeichnet. Zu diesem Zweck werden Bilder der behandelten Bereiche aufgenommen und anhand der Färbung der Oberfläche gruppiert (gelabelt). Die entsprechenden elektrischen Daten (d.h. die von ihnen gebildeten Muster) werden verwendet, um die verschiedenen Gruppen oder Labels zu trainieren (die später als Effektstufen auf der Benutzeroberfläche ausgewählt werden konnten). Die Farbe des behandelten Gewebes wurde gewählt, weil dies die Information ist, die dem Arzt während des Eingriffs zur Verfügung steht. Aufgrund der verwendeten Geräte (System bestehend aus Endoskop, Videoprozessor, Monitor usw.) kann sich die Farbe des Gewebes bei bestimmter Behandlungsdauer von System zu System ändern und ist nicht vergleichbar. Bei der Aufnahme der Bilder werden aber definierte Einstellungen verwendet, um sicherzustellen, dass die Farbgebung der Bilder nicht durch die verwendete Ausrüstung (z. B. Kamera) verfälscht wird und die Ergebnisse vergleichbar sind.

Für die Verwendung des Steuermoduls 19 sind keine Bilder erforderlich, sondern es werden nur die elektrischen Daten zur Vorhersage der Gewebedevitalisierung verwendet.

Die KI-gesteuerte Argon-Plasma-Koagulation ist nur ein Beispiel für die Implementierung einer KI-Kontrollfunktion in ein Modul, in dem ein vorab trainierter (nicht veränderbarer) KI-Algorithmus die Leistungsabgabe steuert. Das Training eines Steuermoduls 19 auf der Grundlage von Labels, bei dem visuelle Daten von echtem Gewebe mit den entsprechenden elektrischen Daten verglichen werden, kann für verschiedene Modi/Indikationen verwendet werden, z. B. Schrumpfung (visuell) für die Qualität der Thermofusion, Grad der Devitalisierung (visuelle Koagulationszone, Kollateralschäden) beim elektrochirurgischen Schneiden von Gewebe, Eindringtiefe (z.B. bei ESD), Vergrößerung von HF-Ablationszonen (visuelle Ablationszone) und elektrische Daten beim elektrochirurgischen Schneiden.

Darüber hinaus ist diese Methode nicht auf elektrische Daten beschränkt, sondern kann auch auf weitere Daten ausgedehnt werden, z.B. zur Kontrolle der Kissenbildung in der Hydrotechnik, Temperaturregulierung bei RFA oder der Eisballbildung in der Kryotechnik.

Nach Umschalten in den zweiten Mode mit niedriger Leistung können die elektrischen Größen der Sensoren S1, S2, S3 geänderte Werte annehmen und sich im Zeitverlauf wieder ändern, wie in Figur 4 durch gepunktete Kurvenäste veranschaulicht ist. Beispielsweise kann der elektrische Strom auf Sensor S1 durch Reduktion der Leistung zunächst abnehmen, durch Rückfeuchtung des Gewebes im Zeitverlauf jedoch wieder leicht ansteigen. Ebenso können sich die durch die anderen Sensoren S2, S3 überwachten Größen im Zeitverlauf wieder ändern. Auch die so erzeugten Muster können in den Trainingsdatensatz einfließen und somit dem Steuermodul 19 zur Verifikation des erreichten Effekts entsprechend dem gewünschten Effekt-Label dienen.

### Bezugszeichen:

- 10, 10': Generator
- 11: Instrument
- 12: Leitung
- 13: Ausgänge des Generators 10
- 14: Neutralelektrode
- 15: Elektrode
- 16: Entladung
- 17: biologisches Gewebe
- 18: Quelle
- 19: Steuermodul mit KI
- 20: Sensorblock
- 21, 22: Pfeil
- 23: KI-Modul
- 24: Eingabeeinrichtung
- 24': Eingabemittel
- 25: Kamera
- 26: Block
- 27: Trainingsdatensatz
- 29: Kurve
- 30: Toleranzfeld-Kästchen in Figur 4
- S1 - S3: Sensoren
- g1 - g3: elektrische Größen
- e1 - e3: Eingänge Generator
- L1 ... L10: Effektlabels
- v: Bilddaten
- M: Muster der elektrischen Größen g1 - g3
- t: Zeit
- tₘ: Messzeitpunkt

## Patentansprüche

1. Generator
mit einem Steuermodul (19) mit Eingängen (e1, e2, e3), an die nur Sensoren (S1, S2, S3) für elektrische Größen (g1, g2, g3) angeschlossen sind,
mit einer elektrischen Quelle (18), die mit dem Steuermodul (19) verbunden und von diesem steuerbar ist und die mit einem medizinischen Instrument (11) verbunden ist, um dieses mit elektrischer Leistung zu versorgen,
wobei das Steuermodul (19) einen Steuerdatensatz (26) umfasst, der auf einem Trainingsdatensatz (27) basiert, der Bilddaten (v) und elektrische Größen (g1, g2, g3) umfasst, die von den Sensoren (S1, S2, S3) erfasst werden.

2. Generator nach Anspruch 1, **dadurch gekennzeichnet, dass** das Steuermodul (19) dazu eingerichtet ist, die Quelle (18) alternativ in einem ersten Mode (HIGH) oder in einem zweiten Mode (LOW) zu betreiben, wobei die Quelle (18) in dem ersten Mode (HIGH) eine hohe und in dem zweiten Mode (LOW) eine niedrige Leistung liefert.

3. Generator nach Anspruch 2, **dadurch gekennzeichnet, dass** die elektrische Leistung des Generators (10) in dem ersten Mode (HIGH) zur Erzielung einer Devitalisierung und Koagulation von biologischem Gewebe (17) bemessen ist und dass die elektrische Leistung des Generators (10) in dem zweiten Mode (LOW) zur Vermeidung einer Devitalisierung und Koagulation von biologischem Gewebe (17) bemessen ist, jedoch so groß, um eine stabile Plasmazündung und eine valide Datenerhebung zu sichern.

4. Generator nach einem der vorstehenden Ansprüche, **dadurch gekennzeichnet, dass** der Steuerdatensatz (26) durch maschinelles Lernen auf Basis des Trainingsdatensatzes (27) mit Bilddaten (v) erzeugt ist.

5. Generator nach einem der vorstehenden Ansprüche, **dadurch gekennzeichnet, dass** die Bilddaten (v) Einzelbilder, Bildsequenzen oder Videodaten sind und dass die elektrischen Größen (g1, g2, g3) als Einzelmesswerte, mehrere in zeitlichen Abständen gewonnene Messpunkte oder zeitliche Verläufe der elektrischen Größen (g1, g2, g3) sind.

6. Generator nach Anspruch 4 oder 5, **dadurch gekennzeichnet, dass** der Steuerdatensatz (26) aus manueller Begutachtung von Gewebeprobebehandlungsresultaten gewonnene Effektlabel (L1 ... L10) enthält.

7. Generator nach Anspruch 5 oder 6, **dadurch gekennzeichnet, dass** der Steuerungsdatensatz (26) Effektlabel (L1 ... L10) enthält, die verschiedene Grade der Gewebedevitalisierung kennzeichnen.

8. Generator nach Anspruch 7, **dadurch gekennzeichnet, dass** das Steuermodul (19) dazu eingerichtet ist, bei Erkennung eines Musters der elektrischen Größen (g1, g2, g3), dem ein gewünschtes Effektlabel (L1 ... L10) zugeordnet ist, von dem ersten Mode (HIGH) in den zweiten Mode (LOW) umzuschalten.

9. Generator nach Anspruch 8, **dadurch gekennzeichnet, dass** das Steuermodul (19) dazu eingerichtet ist, das Muster (M) der elektrischen Größen (g1, g2, g3) nach dem Umschalten in den zweiten Mode (LOW) weiterhin zu überwachen.

10. Generator nach Anspruch 9, **dadurch gekennzeichnet, dass** das Steuermodul (19) darauf eingerichtet ist, von dem zweiten Mode (LOW) in den ersten Mode (HIGH) zu schalten, wenn das im zweiten Mode (LOW) aufgenommene Muster (M) einem zu geringen Effektlabel (L1 ... L10) entspricht.

11. Generator nach Anspruch 9 oder 10, **dadurch gekennzeichnet, dass** das Steuermodul (19) darauf eingerichtet ist, von dem zweiten Mode (HIGH) in einen dritten Mode (off) zu schalten, wenn das im zweiten Mode (LOW) aufgenommene Muster einem zu hohen Effektlabel (L1 ... L10) entspricht.

12. Generator nach einem der vorstehenden Ansprüche, **dadurch gekennzeichnet, dass** das Steuermodul (19) eine Abstandsmessfunktion aufweist.

13. Generator nach einem der vorstehenden Ansprüche, **dadurch gekennzeichnet, dass** das Steuermodul (19) darauf eingerichtet ist, den Abstand zwischen dem Instrument (11) und einem biologischen Objekt (17) anhand der von den Sensoren (S1, S2, S3) erfassten elektrischen Größen (g1, g2, g3) zu ermitteln.

14. Generator nach Anspruch 13, **dadurch gekennzeichnet, dass** das Steuermodul (19) darauf eingerichtet ist, den Abstand anhand der Nichtlinearität der an dem Ausgang (13) des Generators (10) wirksamen Last zu ermitteln.

15. Verfahren zur Gewinnung eines Steuerungsdatensatzes (26) eines Steuermoduls (19) eines elektrochirurgischen Generators (10) und Verfahren zum späteren Betrieb eines solchen Generators (10), bei dem:
in einem Trainingsablauf ein Trainingsdatensatz (27) generiert wird, indem mit dem Generator (10`) in vorgegebener Einstellung auf biologisches Gewebe (17) eingewirkt wird und dabei vorhandene oder sich ergebende elektrische Größen (g1, g2, g3) sowie sich ergebende Gewebeveränderungen mittels Kamera (25) aufgezeichnet werden,
den Gewebeveränderungen Effektlabels (L1 ... L10) zugeordnet werden,
aus dem Trainingsdatensatz (27) durch maschinelles Lernen ein Steuerdatensatz (26) ermittelt wird, der einen Zusammenhang zwischen den elektrischen Größen (g1, g2, g3) und den Effektlabels (L1 ... L10) repräsentiert,
der Generator (10) in einem Anwendungsablauf zur Erzielung eines gewählten, einem Effektlabel (L1 ... L10) entsprechenden Effekts anhand des Steuerdatensatzes (26) gesteuert wird.
